# EUROPEAN PATENT SPECIFICATION

(11) **EP 2 964 100 B1**
(45) Date of publication and mention of the grant of the patent: **20.06.2018**
(21) Application number: 14760678.4
(22) Date of filing: 04.03.2014
(51) Int. Cl.: A61B 8/12, A61M 25/09, A61B 18/00, A61B 8/08, A61B 8/00

(54) **ECHOLUCENT GUIDEWIRE TIP**
ECHOTRANSPARENTE FÜHRUNGSDRAHTSPITZE
POINTE DE FIL-GUIDE ÉCHOTRANSPARENTE

(30) Priority: 06.03.2013 US 201361773199 P
(43) Date of publication of application: 13.01.2016
(60) Divisional application: 18171694.5
(73) Proprietor: Muffin Incorporated, West Lafayette, IN 47906 (US)
(72) Inventor: FISCHER, Frank, J., Bloomington, IN 47401 (US); HAWKINS, Kem, Bloomington, IN 47401 (US); FEARNOT, Neal, E., West Lafayette, IN 47906 (US)
(74) Representative: Garratt, Peter Douglas
(86) International application number: PCT/US2014/020374
(87) International publication number: WO 2014/138099

(56) References cited:
- EP-A2- 0 812 600
- WO-A1-96/13295
- WO-A1-96/19255
- ES-T3- 2 274 539
- JP-A- 2010 188 190
- KR-A- 20110 083 528
- US-A- 5 624 396
- US-A1- 2002 043 113
- US-A1- 2009 292 225
- US-A1- 2010 168 649

## Description

### BACKGROUND

Ultrasound technology has been used for therapeutic and diagnostic medical procedures, which can include providing imaging of internal portions of a body. For example, devices have been proposed for ultrasound imaging within blood vessels to view the condition of the vessel and/or placement or condition of a device placed in the vessel, as well as to help to determine plaque volume and the degree of stenosis within an artery lumen. That information is often difficult to obtain through angiographic imaging and exterior ultrasound imaging, particularly in regions having multiple overlapping arterial segments.

Typically, a catheter is fitted with a transducer at an end. A guidewire is positioned within a body conduit through use of angiography or ultrasound. The catheter is slid over the guidewire and positioned near the farthest end of the guidewire at the farthest end of the body conduit which is to be imaged. The transducer transmits ultrasonic sound waves and receives the reflected sound wave information. Existing transducers typically transmit sound waves either through the side of the transducer or through the forward face of the transducer. Forward-facing transducers can be preferable because they generally provide information useful in guiding the catheter forward safely.

It is advantageous for guidewires to be echogenic (or ultrasonically visible) during insertion so that the physician can image the guidewire from outside the patient in order to optimally place the guidewire in the body conduit. It is also advantageous that the guidewire have sufficient stiffness in order to be properly manipulated to the imaging site. However, the echogenicity of the guidewire can become problematic when used with forward-facing transducer, due to the reflection of ultrasonic waves by the wire. For example, as a forward-facing transducer is retracted along the guidewire, the guidewire reflects the sound waves and partially blocks the view of the transducer, thus impairing the forward viewing capabilities of the catheter.

Thus, there is a need for guidewires which have sufficient stiffness for initial placement and guiding of forward facing transducer and which do not interfere with the ultrasound signal. WO 96/13295 discloses an infusion guidewire or catheter for introduction through a selected path in a patient's vascular system to a site in a blood vessel for infusing a drug or agent into the blood vessel. EP0812600 discloses a guidewire having an elongate core with a distal and proximal end and a coiled spring surrounding the distal end of the core wire. ES2274539 relates to intravascular paraguage wires, in particular those with improved flexibility and radiological opacity properties. US5624396 discloses a system for use in delivering thrombolytic agent to a firm obstruction in a blood vessel. The distal infusion segment assumes a pre-biased J-shape bend configuration when positioned. The device comprises a spring loaded assembly between the proximal ends of the device. WO 96/19255 relates to a drug delivery device having a hollow tube which is configured to serve as a guide wire for intraluminal procedures.

### SUMMARY

Essential features of the invention are set out in the independent claim. Optional features are set out in the dependent claims. There are disclosed embodiments of a system for use with ultrasound procedures including a guidewire having an atraumatic end and a fluid path at least partially within the atraumatic end, and a support member or mandrel at least partially within and retractable with respect to the fluid path. The fluid path has in use a first configuration in which the portion of the fluid path within the atraumatic end is substantially filled with gas such that the end has enhanced echogenicity and a second configuration in which the portion of the fluid path within the atraumatic end is substantially filled with liquid such that the end is substantially echolucent. In particular examples, a catheter can be provided, having a lumen for accommodating the guidewire, so that the catheter can move along the guidewire. The catheter can include a probe for use with an ultrasound system. The fluid path may be a closed path connected to a liquid or gas source. An end cap is positioned at a distal end of the medical device in some embodiments, wherein the end cap is attached to a support tube. The end cap can include a shoulder which engages an application side end of the support tube, and/or be constructed of an echolucent polymer material which is substantially echolucent when positioned in a body conduit. Examples of the support member or mandrel can include a tapered end and/or be constructed of a polymer material.

Methods are also disclosed, including a method including inserting a guidewire having an atraumatic end and an optional fluid path at least partially within the atraumatic end into a body conduit, moving an intravascular ultrasound probe over the guidewire within the body conduit, and altering an ultrasound characteristic of at least a portion of the guidewire while the portion of the guidewire is within the body conduit. The altering can include removing a support member from at least a portion of the fluid path within the atraumatic end. In particular examples, the altering includes removing one of a liquid or gas from at least a portion of the fluid path within the atraumatic end, and adding one of liquid or gas to at least a portion of the fluid path within the atraumatic end. The altering can result in at least a portion of the atraumatic end being substantially echolucent, or in at least a portion of the atraumatic end being echogenic (e.g. easily observable within a body conduit). Following the altering, the user can retract the ultrasound probe with respect to the guidewire, wherein imaging by the ultrasound probe occurs without significant interference by the atraumatic end of the guidewire. The procedure can be repeated. For example, following the retracting of the ultrasound probe, the user can retract the guidewire so that the atraumatic end is adjacent or within the ultrasound probe, and can repeat the step of retracting the ultrasound probe with respect to the guidewire, wherein imaging by the ultrasound probe occurs without significant interference by the atraumatic end of the guidewire.

In other examples, a medical device can include a catheter having a wall with an inner surface defining a central longitudinal lumen and an outer surface, and a plurality of separate lumens positioned within the wall between the inner surface and the outer surface, with the plurality of separate lumens oriented radially with respect to each other and extending parallel to and offset from the central longitudinal lumen. The plurality of separate lumens have in use a first configuration in which the lumens are substantially filled with liquid such that the catheter has reduced echogenicity when positioned in a body conduit and a second configuration in which the lumens are substantially filled with gas such that the catheter has enhanced echogenicity when positioned in a body conduit. In particular examples, the plurality of separate lumens are closed fluid paths.

In other embodiments, a system for use with ultrasound procedures includes a guidewire having an atraumatic end portion and a lumen. The end portion has an end cap and the lumen extends to the end cap. A support member is at least partially within the lumen and movable between a first position in which a portion of the support member is within the end portion and a second position in which the support member is not within the end portion. Retracting the support member alters the echogenicity of the end portion when the end portion is positioned within a body conduit. In some embodiments, the system includes a hole in the end cap, such that repositioning of the support member from the first position to the second position causes liquid from the body conduit to move through the hole into the end cap.

In other embodiments, a system for use with ultrasound procedures includes a guidewire having a tip portion and a body portion. The tip portion is constructed of a polymer which is echo lucent when placed in the body conduit and the body portion is echogenic when placed in a body conduit.

The medical devices described herein address the issues mentioned above as well as others. For example, examples of a guidewire for medical applications are disclosed which includes a guidewire with a lumen, a plastic tip, and a removable mandrel. The guidewire has echogenic properties which can be altered between an echogenic state and an echolucent state by evacuating the lumen and applying a liquid or gas within the lumen. In this way, the guidewire can be positioned within a body conduit through use of ultrasound while configured in an echogenic state. Similarly, the guidewire can be configured in an echolucent state during a medical procedures in order to avoid impairing the forward viewing capabilities of an ultrasound probe. A catheter with a number of lumens can be altered between an echogenic and echolucent state.

### BRIEF DESCRIPTION OF THE DRAWINGS

FIG. 1 illustrates a perspective view of a medical device and guidewire positioned within a body conduit.
FIG. 2 illustrates a cross-sectional view of an end of the guidewire of FIG. 1 with a support member inserted.
FIG. 3 illustrates a cross-sectional view of an end of the guidewire of FIG. 2 having a support member removed and filled with a liquid.
FIG. 4 illustrates a cross-sectional view of an end of the guidewire of FIG. 2 having a support member inserted and filled with a liquid.
FIG. 5 illustrates a partial cross-sectional perspective view of a portion of a catheter having lumens extending therethrough.

### DESCRIPTION OF THE SELECTED EMBODIMENTS

For the purpose of promoting an understanding of the principles of the disclosure, reference will now be made to the embodiments illustrated in the drawings, and specific language will be used to describe the same. It will nevertheless be understood that no limitation of the scope of the claims is thereby intended. Any alterations and further modifications in the described embodiments and any further applications of the principles of the disclosure as described herein are contemplated as would normally occur to one skilled in the art to which the disclosure relates. It will be apparent to those skilled in the relevant art that some features that are less relevant to the present disclosure may not be shown for the sake of clarity.

With respect to the specification and claims, it should be noted that the singular forms "a", "an", "the", and the like include plural referents unless expressly discussed otherwise. As an illustration, references to "a device" or "the device" include one or more of such devices and equivalents thereof. It also should be noted that directional terms, such as "up", "down", "top", "bottom", and the like, are used herein solely for the convenience of the reader in order to aid in the reader's understanding of the illustrated embodiments, and it is not the intent that the use of these directional terms in any manner limit the described, illustrated, and/or claimed features to a specific direction and/or orientation.

Referring now generally to the drawings, there are shown exemplary embodiments of a medical device for internal ultrasound procedures. Such devices may be diagnostic or therapeutic (including interventional) in application, and include devices inserted percutaneously, subcutaneously or endoluminally into the patient. The medical device can be used with a system which includes a console (not shown) for processing data or signals received from an ultrasound transducer. The ultrasound console can be a type which is generally used for medical ultrasonic imaging, e.g. generally including control devices usable by a physician and a graphic display which displays graphical images obtained during an ultrasound procedure. The medical device can be used for obtaining images at various locations of a body and/or within a body conduit such as a ducts, tracks, orifices, invaginations, blood vessels, arteries, veins, urethra, ureter, vagina, rectum, throat, ear, nasal, bile duct, esophagus, trachea, or through an artificial tract (or lumen) by percutaneous puncture for example. The console portion can be connected to commercially-available ultrasound probes or catheters with compatible pinout, or other medical devices which are configured for endoluminal procedures. The medical device is capable of transmitting and receiving ultrasound signals and then communicating data obtained from ultrasound signals to the console. The medical device includes a control end which during use is nearest to the user and an application end which during use is nearest to the user's point of interest. The terms "control" and "application" are used throughout this description to describe these positional orientations.

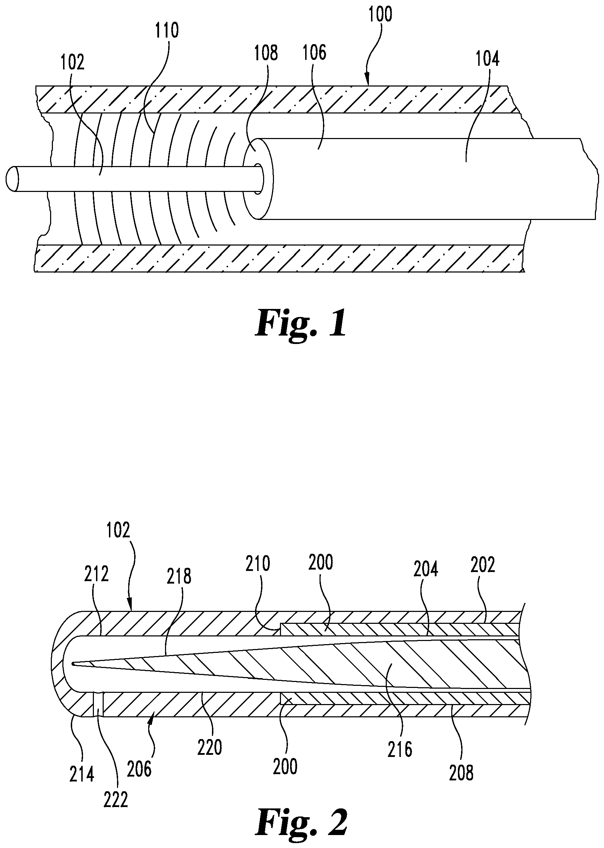

Referring to Figs. 1-5, various embodiments of the present concept are illustrated. FIG. 1 shows a partial perspective view of a medical device located in a body conduit 100. The medical device includes a guidewire 102 and a catheter 104. The catheter 104 includes a probe 106 located at an application end of catheter 104. The probe 106 includes a transducer configured to transmit and receive ultrasound signals through a face 108. The term "transducer" should be understood to include an assembly of two or more parts as well as a single piece. It will further be understood that "transducer" as used herein includes devices (e.g. piezoelectric element(s)) that transmit ultrasound signals (i.e. transform an electrical (RF) signal to ultrasound), receive ultrasound signals (i.e. transform ultrasound to an electrical (RF) signal), or both. If multiple transducers or pieces are provided, transmission of ultrasound may occur at one and reception at another. Transducer(s) as described herein may have one or more piezoelectric elements as respective transducers, and may operate in combination with other transducers within or outside the body. As examples, "transducer" as used herein includes a single element transducer or a one-dimensional array of elements.

The medical device is configured to produce an ultrasound image from within the body conduit 100. The medical device generally works by first inserting the guidewire 102 percutaneously into the body conduit 100 and positioning the guidewire 102 tip at a location at or past the point that represents the furthest application end of the area of interest for imaging by the user. Once the guidewire 102 is positioned, the catheter 104 and probe 106 are positioned onto the guidewire and moved axially along the guidewire until the face 108 is aligned with or slightly past the furthest application end of the area to be imaged. Once the guidewire 102 and the catheter 104 are positioned appropriately, the probe 106 is activated and the catheter 104 is caused to slowly retract along the guidewire 102. The transducer is configured to transmit a forward sensing ultrasound signal 110 through face 108 as depicted in FIG. 1. As the catheter 104 is retracted along the guidewire 102, the probe 106 is turned on or powered so that the transducer transmits an ultrasound signal 110. The signal is partially reflected at the boundaries of various tissues and other elements within the body conduit 100. Part of that signal is reflected back to the transducer through face 108. The probe 106 receives the reflected signal which is used to produce an image through the ultrasound imaging system (not shown). In this way, the ultrasound imaging system can obtain localized and targeted imaging data from within the body conduit 100.

The guidewire 102 includes a support tube 200, a tube end 206, and a support member 216. The guidewire 102 includes an application side end (FIG. 2) and a control side end which is not shown. It should be noted that the embodiment of FIG. 2 is shown for illustrative purposes only and is not to scale. The support tube 200 is a generally tubular shaped object having an outer surface 202 and an inner surface 204. The support tube 200 extends substantially through the length of the guidewire 102. The support tube 200 provides support and controllability to the guidewire 102 during insertion and also during imaging procedures. In some embodiments, support tube 200 is a coil or a braided tube or any of a variety of suitable structures. In some embodiments, the support tube 200 is constructed of metal. In other embodiments, support tube 200 can be constructed wholly or partially from a polymer which is configured to be substantially echolucent in particular environments. For example, partial segments (or axial sections) of support tube 200 can be constructed from metal with the remaining segments constructed from a polymer. In some embodiments, portions of support tube 200 are constructed from multiple concentric polymer layers having varying densities, which provides favorable and simultaneous support and flexibility characteristics.

As used herein, echolucent means having small ultrasound attenuation, or having a small difference in acoustic impedance with a surrounding environment (i.e. body conduit 100). As used herein, echogenic means having a relatively large ultrasound attenuation, or having a large difference in acoustic impedance with a surrounding environment (i.e. body conduit 100).

The illustrated embodiment of guidewire 102 includes a tube end 206 (or end cap, or tip) positioned at the application end (or tip) of the guidewire 102. The tube end 206 is attached to the support tube 200 circumferentially along an inside surface 208 of the tube end 206 such that the inside surface 208 abuts against the outer surface 202 of the support tube 200. This attachment can be friction fit. Alternatively, the attachment can be through use of adhesive, threading, or other suitable method of attachment. The tube end 206 further includes a shoulder 210 which abuts against an end of the support tube 200 such that the tube end 206 extends axially further in the application direction from the application side end of the support tube 200. In this way, the support tube 200 does not extend fully into the application end of the guidewire 102. The coupling of tube end 206 and support tube 200 is contiguous and creates a smooth inside surface which defines an inner lumen 212 (or fluid path). In some embodiments, tube end 206 at its furthest application side end is generally closed such that inner lumen 212 (or fluid path) is fluidly isolated from an exterior surface 214 of the tube end 206. In other embodiments, an optional hole 222 is placed through a side (or tip) of tube end 206 which can allow passage of a liquid or gas therethrough.

The tube end 206 is constructed of a material that presents a minimal barrier to the passage of ultrasound signals so that ultrasound images of surrounding matter (e.g. tissue(s) or implant(s)) may be reasonably acquired through the barrier. For example, materials which are substantially echolucent (i.e. having small ultrasound attenuation, or having a small difference in acoustic impedance with the surrounding environment) when placed in the surrounding working environment, such that the material acts as an acoustic window which allows passage of ultrasound signals with minimal reflection. For example, when used within a blood vessel containing body tissues and blood, it is preferable for tube end 206 to be constructed of a material which has acoustic impedance similar to that of body fluids such as blood. Possible materials could include, for example, a polymer material such as high density polyethelene, polymethylpentene (PMP), or acrylonitrile butadiene styrene (ABS). Accordingly, the tube end 206 could be constructed of any of a variety of suitable materials which are echolucent when immersed in a body conduit.

Guidewire 102 in the illustrated embodiment includes a support member 216 or mandrel. The support member 216 extends throughout the length of the guidewire 102. In some embodiments, support member 216 has a tapered end 218 (FIG. 2). The support member 216 on the control side of the tapered end 218 is substantially cylindrical in shape such that it fits within support tube 200 and lumen 212. In some embodiments, support member 216 has an outer diameter which is less than the diameter of lumen 212, such that the space between support member 216 and the inner wall of lumen 212 acts as a fluid channel. In other embodiments, support member 216 has an outer diameter which is substantially the same as lumen 212 so as to prevent the flow of fluid between the inner wall of the lumen 212 and support member 216. In still further embodiments, support member 216 has an end (i.e. a full end) which is sized to fill lumen 212 and the cavity created by inner surface 212 of tube end 206 (not shown). The support member 216 can be constructed of a metal which is configured to be substantially echogenic in particular environments. In other embodiments, support member 216 can be constructed of a polymer material which is configured to be substantially echogenic in particular environments. In other embodiments, support member 216 can be constructed of a polymer material which is configured to be substantially echolucent in particular environments.

In some examples
and configurations of the medical device, the application side end of the support member 216 is positioned substantially within the tube end 206. At least a portion of the application side end (or tapered end 218) of the support member 216 has resilient and flexible characteristics. Similarly, the tube end 206 is resilient and flexible due to its polymer construction. In some examples, the resilient and flexible characteristics of the application side end of support member 216 in combination with the tube end 206 creates a flexible or pliable atraumatic section of the guidewire 102. In other examples, the combination of the tapered end 218 and the tube end 206 creates a flexible or pliable atraumatic section of the guidewire 102. In the context of this description, atraumatic means a section of the guidewire 102 which is configured to not produce any injury or damage to the inside of a body conduit. As indicated in Figs. 2-4 an example of such an atraumatic section has a blunt end that is rounded or part spherical. In this way, the atraumatic section allows the guidewire 102 to be manipulated and positioned within the body conduit 100 without causing significant or excessive damage to body conduit 100 or tissues located within body conduit 100.

The lumen 212 is further defined by the inner surface 204 of the support tube 200 and the inner surface 220 of the tube end 206. In the illustrated embodiment, the inner diameter of support tube 200 is substantially the same as the inner diameter of tube end 206. The lumen 212 is substantially supported by the support tube 200 such that the support tube 200 causes the lumen 212 to remain open during a medical procedure. Lumen 212 extends through the length of the guidewire 102 in particular embodiments, and is closed on the furthest application side end such that the lumen 212 is not in fluid contact with the exterior of the guidewire 102. In that way, the lumen 212 provides an isolated and closed fluid path which extends throughout the length of the guidewire 102. In other embodiments, a hole 222 is placed through a side (or tip) of tube end 206 which can allow passage of a liquid or gas therethrough. The lumen 212 is connected or connectable to one or more sources or pumps (not shown) which can provide gas and/or liquid to the lumen 212. Similarly, evacuation of lumen 212 of either gas or liquid or both can be performed via lumen 212. For example, the lumen 212 can be filled with a gas and alternatingly the gas can be evacuated and the lumen 212 can be subsequently filled with a liquid. In some examples, one or more holes positioned in a side of guidewire 102 can be used to alternatingly fill lumen 212 with a liquid or gas.

The support member 216 is movable within the lumen 212 and can be retracted through the guidewire 102 either wholly or partially. For example, the support member 216 can be retracted to a point where its furthest application side end is on the control side in relation to the furthest application side end of the support tube 200.

The structure of the application side end of the guidewire 102 including the tube end 206, the support member 216, and the support tube 200 allow the echogenic properties of guidewire 102 to be altered. Generally, an ultrasound signal is partially reflected at the interface of two mediums having different acoustic impedances. The amount of reflection (or attenuation) is a function of the difference between the acoustic impedances of the two mediums. The reflection is stronger when the difference between the acoustic impedances is larger. The acoustic impedance of a gas such as CO₂ is significantly different in this context from the acoustic impedance of a liquid such as blood, while the acoustic impedance of certain polymers is significantly similar to that of liquids such as blood.

According to the embodiments described herein, guidewire 102 (or portions thereof) can be configured in various ways to be alternatingly echogenic and echolucent in body conduit 100. In other words, as described herein, guidewire 102 includes means for alternatingly filling lumen 212 (or fluid path) with liquid and gas. Generally, it is advantageous for the tip of guidewire 102 to be echogenic during placement within body conduit 100 and echolucent during internal ultrasound procedures.

In one exemplary configuration, the guidewire 102 can be configured echogenically with a metal support tube 200 and lumen 212 containing a metal support member 216 with tapered end 218 and a gas. The gas can be CO₂ or any other suitable gas. Because the tube end 206 is constructed of a material having acoustic impedance similar to that of liquids such as blood, a major reflection surface is the interface between the gas and inner surface 220 of the tube end 206 during insertion and positioning of guidewire 102. Another major reflection surface is the interface between the gas and the outer surface of metal support member 216. In this way, the gas causes the guidewire 102 and specifically the application side end of the guidewire 102 in at least the region of tube end 206 to be echogenic when surrounded by liquids such as blood in body conduit 100. Because the tip of guidewire 102 in this configuration is echogenic, the tip can be positioned within the vasculature using traditional techniques with the assistance of exterior ultrasound. For increased structural support, the support member 216 can be positioned within the tube end 206 according to the configuration shown in FIG. 2 with a gas taking up the space between the support member 216 and the surface of the lumen 212. In this way, the application side tip of the guidewire 102 is imageable through ultrasound procedures during insertion within a body. This allows the guidewire 102 to be accurately positioned within the body and more specifically at a specific location within a body conduit 100.

The tip of guidewire 102 can be configured echolucently when positioned in body conduit 100 by removing support member 216 from tube end 206 and adding a liquid to lumen 212. Support member 216 can be fully or partially removed from lumen 212. The liquids described herein are any of a number of liquids or gels which are safe and suitable for medical purposes and having suitable acoustic impedance (e.g. saline, oils, alcohols, or blood). With support member 216 removed from tube end 206 and tube end 206 containing a liquid, tube end 206 becomes echolucent when positioned within body conduit 100. In some embodiments, the liquid can be added by first evacuating the gas from lumen 212 (e.g. by pumping) and adding a liquid. The echolucent properties of the tip of guidewire 102 are due to the material properties of the tube end 206, the liquid, and the support member 216 having acoustic impedance similar to that of the surrounding blood or other fluid in body conduit 100. Due to the similarity of the acoustic impedances, in this embodiment, there are very few if any ultrasonically reflective surface interfaces within the application side end of guidewire 102.

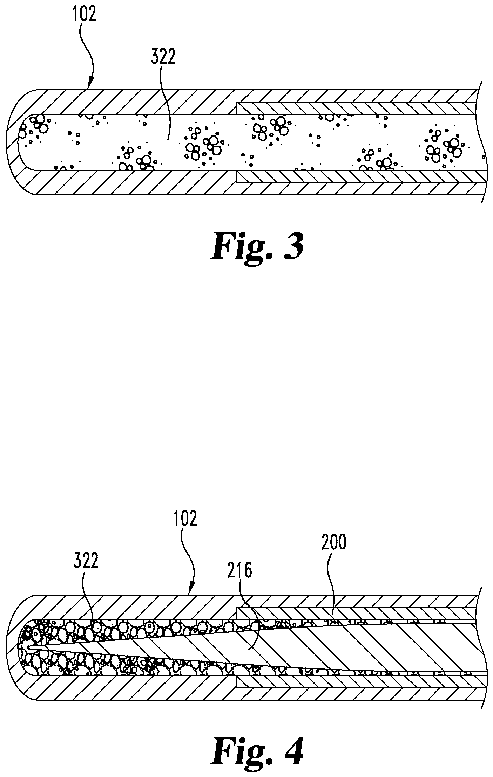

During an exemplary procedure, the guidewire 102 is configured to be echogenic according to the above exemplary configuration (i.e. with a gas in lumen 212, metal support member 216 with tapered end 218, and metal support tube 200). Guidewire 102 is positioned within the vasculature using traditional techniques (e.g. external ultrasound). It is advantageous for the position to be one in which tube end 206 is at the furthest application side end of the area of interest for imaging. Once the guidewire 102 is positioned, the catheter 104 is placed over the guidewire 102 and inserted along the guidewire 102 to a point where the probe 106 is at or just past the furthest application side end of the area to be viewed, e.g. at or beyond the blunt end of tube end 206. In other words, the transducer (and face 108) is positioned such that it is able to transmit ultrasound waves which can be reflected off of tissues which are to be imaged. Once the probe 106 is positioned the support member 216 can be removed from at least a portion of the tube end 206. In some examples, support member 216 could also be removed from the entire length of the guidewire 102. The tube end 206 is then made echolucent by evacuating the gas from lumen 212 and replacing it with a suitable liquid 322, as shown in FIG. 3.

Once the guidewire 102 is configured in an echolucent state, probe 106 is mechanically or manually moved (e.g. retracted) along the guidewire 102. As the probe 106 is slowly retracted, the transducer transmits an ultrasound signal generally towards the application side of the medical device (i.e. rearward or opposite relative to the direction of motion of probe 106). The ultrasound signal travels until it encounters an acoustic impedance boundary (e.g. body tissue, plaque, medical implant, or other material which has acoustic impedance sufficiently different from bodily fluids or other surrounding material) such that the ultrasound signal is at least partially reflected at the boundary. At least a portion of the ultrasound signal is reflected back towards the face 108. One or more electrical signals representing reflected ultrasound received at the transducer via face 108 are sent from probe 106 via a conduction pathway to the ultrasound console for imaging and/or other data display to the physician. Simultaneously or subsequently, the transducer continues to transmit further ultrasound signals and the process is repeated, in certain examples over a desired period of time.

During the ultrasound procedure, because the tube end 206 is at least substantially echolucent within the body conduit 100 when exposed to the ultrasound signal from the probe 106, the guidewire 102 produces little or no interference or reflection (e.g. dark images or artifacting) within the ultrasound signal due to reflections from the tube end 206. Retraction of catheter 104 is continued until probe 106 is at or near the application side end of the support tube 200. Once the probe 106 reaches that point, the imaging procedure can be halted or interrupted and the guidewire 102 can be retracted within body conduit 100 such that the application side end of the guidewire is at a location closer to or within the probe 106 or catheter 104. At that time, the ultrasound procedure can resume as the catheter 104 is further retracted such that the probe 106 moves along the tube end 206 portion of the guidewire 102. When sufficient data is obtained, the catheter 104 and the guidewire 102 can be withdrawn from the body conduit 100.

Various alternative configurations and embodiments according to the principles discussed herein are within the scope of this disclosure. Variations include but are not limited to combinations of the following: support member 216 being constructed of a metal or polymer material and having a tapered end 218 or a full end (not shown); support tube 200 being constructed of a metal material, a polymer material, or segmented sections including a mix of both; the optional fluid channel can contain gas in an echogenic state or a liquid in an echogenic or echolucent state; and an optional hole 222 positioned near the tip of guidewire 102 for passage of a liquid or gas therethrough.

In an alternative configuration of the guidewire 102 depicted in FIG. 4, the support member 216 is constructed of HDPE (high density polyethylene) or other suitable polymer material which is packed in a gel or a liquid 322, so that the combination has acoustic impedance similar to that of blood. Because the support member 216 is constructed of a plastic material having acoustic impedance similar to that of the liquid 322 and of blood, the tube end 206 of the guidewire 102 is at least substantially echolucent in body conduit 100. The guidewire 102 can be altered from an echolucent state to an echogenic state by evacuating the liquid 322 and/or introducing a gas such as CO₂, for example through use of the control side end of guidewire 102 and a pump. As discussed previously, the difference in impedance between the gas and the material of the tube end 206 causes the tube end 206 to be echogenic when positioned in a body conduit. According to the embodiment of FIG. 4, the support member 216 does not need to be removed from the tube end 206 in order for the tube end 206 to be configured in an echolucent state. In that way, by having the ability to replace the liquid 322 with a gas or otherwise change ultrasound characteristics, the guidewire 102 and more specifically the tube end 206 is able to alternate between echolucent and echogenic configurations.

In another alternative configuration, the tip of guidewire 102 can be configured echogenically with a metal support tube 216 and lumen 212 containing a metal support member 216 with tapered end 218 and a liquid. Because the tube end 206 is constructed of a material having acoustic impedance similar to that of liquids such as blood, a major ultrasound reflection surface is the interface between support member 216 and the liquid during insertion and positioning of guidewire 102. In this way, the metal support tube 200 causes the guidewire 102 and specifically the application side end of the guidewire 102 in at least the region of the tube end 206 to be echogenic when surrounded by liquids such as blood in body conduit 100. The tip of guidewire 102 can be configured echolucently when positioned in body conduit 100 by removing support member 216 from tube end 206 and adding additional liquid to lumen 212. Support member 216 can be fully or partially removed from lumen 212. With support member 216 removed from tube end 206 and tube end 206 containing a liquid, tube end 206 becomes echolucent when positioned within body conduit 100.

In another alternative configuration, the tip of guidewire 102 can be configured echogenically with a metal or polymer support tube 200 and lumen 212 containing a polymer support member 216 with tapered end 218 and a gas. Because support member 216 is constructed of a plastic material having acoustic impedance significantly different from that of gas, the tip of guidewire 102 is relatively echogenic in body conduit 100. A major reflection surface is the interface between the gas and inner surface 220 of tube end 206. Another major reflection surface is the interface between the gas and the outer surface of polymer support member 216. Guidewire 102 can be altered from an echogenic state to an echolucent state by evacuating the gas and introducing a liquid, e.g. through use of the control side end of guidewire 102 and a pump.

In another alternative configuration (not shown), the tip of guidewire 102 can be configured echogenically with a metal or polymer support tube 200 and lumen 212 containing a metal support member 216 with a full end and no fluid channel. In this configuration, support member 216 completely fills lumen 212. A hole 222 (e.g. FIG. 2) is positioned in the side of tube end 206. Because support member 216 is constructed of a metal having acoustic impedance significantly different from that of the material of tube end 206, the tip of guidewire 102 is relatively echogenic in body conduit 100. The major reflection surface is the interface between the inner surface 220 of tube end 206 and the outer surface of support member 216. Guidewire 102 can be altered from an echogenic state to an echolucent state by withdrawing support member 216. As support member 216 is withdrawn from tube end 206, a pressure differential is created between the interior and the exterior of tube end 206. The pressure differential causes blood or other fluid located in body conduit 100 to pass through hole 222 and fill the interior of tube end 206 until the pressure is equalized. Tube end 206 filled with blood or other fluid is substantially echolucent in body conduit 100. The tip of guidewire 102 can be made echogenic again by moving support member 216 back into tube end 206 and causing blood or other fluid to flow out of tube end 206 through hole 222. In that way, the tip of guidewire 102 is alternatingly echogenic and echolucent when positioned in body conduit 100.

In another alternative configuration (not shown), the tip of guidewire 102 can be configured echogenically with a metal or polymer support tube 200 and lumen 212 containing a metal support member 216 with tapered end 218 and a liquid. A hole 222 (FIG. 2) is positioned in the side of tube end 206. Because support member 216 is constructed of a metal having acoustic impedance significantly different from that of the material of tube end 206 and the liquid, the tip of guidewire 102 is relatively echogenic in body conduit 100. The major reflection surface is the interface between support member 216 and the inner surface 220 of tube end 206. Guidewire 102 can be altered from an echogenic state to an echolucent state by withdrawing support member 216. As support member 216 is withdrawn from tube end 206, a pressure differential is created between the interior and the exterior of tube end 206. The pressure differential causes blood or other fluid located within body conduit 100 to pass through hole 222 and mix with the liquid in lumen 212 while filling the void created in the interior of tube end 206 until the pressure is equalized. Tube end 206 filled with a mix of blood and liquid is substantially echolucent in body conduit 100. The tip of guidewire 102 can be made echogenic again by moving support member 216 back into tube end 206 and causing a mixture of blood and the liquid to flow out of tube end 206 through hole 222. In that way, the tip of guidewire 102 is alternatingly echogenic and echolucent when positioned in body conduit 100.

Any of the configurations described herein could include a segmented support tube 200 as described previously, wherein particular segments of guidewire 102 can be configured to be permanently or alternatingly echogenic and/or echolucent when positioned in body conduit 100. For example, support tube 200 includes polymer segments having acoustic impedance configured to be substantially echolucent when lumen 212 contains a liquid and when in body conduit 100. The polymer segments can be interspersed amongst other segments constructed of metal or polymer having acoustic impedance configured to be substantially echogenic in body conduit 100. In such a configuration, particular segments of guidewire 102 having polymer segments of support tube 200 are alternatingly echogenic or echolucent (depending on whether lumen 212 is filled with a gas or a liquid, and the material construction of support member 216) when positioned in body conduit 100.

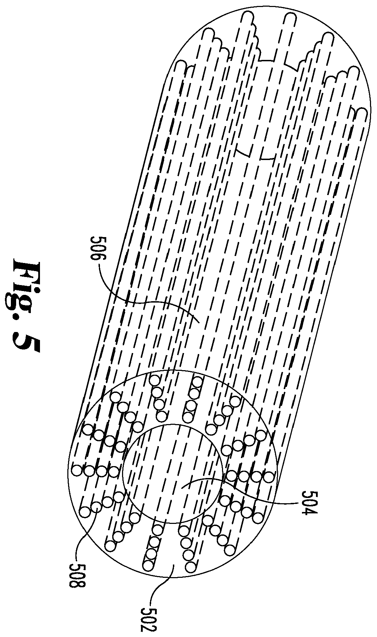

A further alternative example shown in FIG. 5 is a partial cross-sectional perspective view of a portion of a catheter 500. The catheter 500 has a wall 502 with an inner surface 504 (defining a central longitudinal lumen) and an outer surface 506. The catheter 500 has a plurality of lumens 508 positioned within the wall between the inner surface 504 and the outer surface 506. In the illustrated example, the lumens 508 extend longitudinally substantially through the length of the catheter 500, with an end terminating near the application side end of the catheter 500 (not shown). The illustrated example includes a number of respective sets of lumens each in radial relation with each other (i.e. each lumen of a particular set lies along a radius of catheter 500) and offset from the central lumen. The respective sets of lumens are substantially evenly spaced around the circumference of catheter 500 in this particular example, and pair(s) of such sets may be linearly aligned across the central lumen from each other.

Lumens 508 may be alternatingly filled with gas or liquid to alter the ultrasound characteristics of catheter 500. For example, the control side end of the lumens 508 can be connected to a source (not shown) which can evacuate the lumens 508 and/or fill them with a gas or a liquid. The gas or liquid can be those mentioned previously, such as CO₂ or saline as examples, among others. The lumens 508 can have the same diameter or they can be differently sized. The lumens 508 can be in an ordered state as exemplified above or alternatively be positioned randomly within the catheter 500. When the lumens 508 are filled with a liquid (e.g. saline) having ultrasound characteristics like blood and the material of catheter 500, the catheter 500 has a substantially echolucent characteristic when positioned within a body conduit according to the principles discussed herein. When the lumens 508 are filled with a gas, the catheter 500 has a substantially echogenic characteristic when positioned within a body conduit. In this way, a catheter 500 is provided in which the echogenicity can be altered from an echogenic state to a less echogenic (or echolucent) state.

While some of the above discussion concerned specific use in the context of ultrasound system applications, it will be understood that embodiments of medical devices described herein could also be used for a variety of other medical procedures and with a variety of other medical devices. The versatility of the embodiments described herein allows the devices to be used for percutaneous therapeutic interventions such as, for example, embolism coils, stents, filters, graphs, balloons, biopsies, and ministering therapeutics, etc. the devices can be used to locate various anatomical landmarks that will be used to correctly place or guided therapy. Typical landmarks include confluences, bifurcations, side branches, nearby vessels, nearby nerves, the heart, and other tissues adjacent to vessels or other orifices containing the transducer. The devices can also be used for procedures such as locating diseased tissue that will be treated or avoided. During a TIPS (transjugular intrahepatic portocaval shunt) procedure an image can be produced to allow a physician to watch a needle being placed into the portal vein. For AAA (aortic abdominal aneurysm) graft delivery, the devices can allow a physician to place a guidewire into a contralateral leg. The devices could also be used to image the location of a deployed implantable device both during and after deployment.

Although particular materials were highlighted herein for some components of the devices, those materials are not intended to be limiting of the types of materials which are suitable to be used. Additionally, where materials were not highlighted, a variety of materials could be used such as certain types of metals, polymers, ceramics or other types of materials which are suitable for use in devices for small body cavity applications. Where polymer was specified as a material for support tube 200, support member 216, and tube end 206, the specific polymer can be chosen from a variety of polymers that have favorable acoustic impedance properties. In some cases, the type of material can vary depending on the acoustic properties of the particular body conduit for which the device is intended.

While the disclosure has been illustrated and described in detail in the drawings and foregoing description, the same is to be considered as illustrative and not restrictive in character, it being understood that only particular embodiments have been shown and described and that all changes, equivalents, and modifications are desired to be protected. Significantly, the drawings are not drawn to scale and should not be taken as such; rather the drawings are merely for illustrative purposes only and some features and aspects are exaggerated or diminished in order to further understanding of the invention described herein. Aspects or features particularly shown or described with respect to one embodiment or example may be incorporated into other embodiments, features or examples. Further, although the particular embodiments discussed herein have been noted in the context of use in blood vessels, it will be understood that embodiments may also be used in other bodily locations or environments.

## Claims

1. A system for use with ultrasound procedures comprising:
a guidewire (102) having an atraumatic end and a portion of a fluid path at least partially within the atraumatic end;
a support member (216) at least partially within and retractable with respect to the fluid path; **characterized in that** the fluid path has in use a first configuration in which the portion of the fluid path within the atraumatic end is substantially filled with gas such that the end has enhanced echogenicity and a second configuration in which the portion of the fluid path within the atraumatic end is substantially filled with liquid such that the end is substantially echo lucent.

2. The system of claim 1, further comprising a catheter (104) having a lumen sized to accommodate the guidewire, so that the catheter is movable along the guidewire.

3. The system of claim 2, wherein the catheter includes a probe (106) for use with an ultrasound system.

4. The system of claim 1, wherein the fluid path is a closed path connected to a liquid or gas source.

5. The system of claim 1, further comprising an end cap positioned at an application side end of the guidewire, wherein the end cap is attached to a support tube (200).

6. The system of claim 5, wherein the end cap includes a shoulder which engages an application side end of the support tube.

7. The system of claim 5, wherein the end cap is constructed of a polymer material which is substantially echolucent when positioned in a body conduit.

8. The system of claim 1, wherein the support member includes a tapered end (218).

9. The system of claim 1, wherein the support member is constructed of a polymer material.

10. The system of claim 5, further comprising a hole in the side of the end cap.

11. The system of claim 5, wherein the support tube is a coil or a braided tube.

12. The system of claim 5, wherein the support tube is constructed of metal.

13. The system of claim 5, wherein the support member has an outer diameter which is less than the diameter of the fluid path.

14. The system of claim 5, wherein the support member has an outer diameter substantially the same as the fluid path.

15. The system of claim 5, wherein the support member is at least partially within the fluid path and movable between a first position in which a portion of the support member is within the end portion and a second position in which the support member is not within the end portion; and
wherein retracting the support member alters the echogenicity of the end portion when the end portion is positioned within a body conduit.

## Patentansprüche

1. System zur Verwendung mit Ultraschallverfahren, das aufweist:
einen Führungsdraht (102) mit einem atraumatischen Ende und einem Teil eines Fluidwegs, der sich wenigstens teilweise in dem atraumatischen Ende befindet;
ein Trägerelement (216), das wenigstens teilweise in dem Fluidweg und in Bezug darauf zurückziehbar ist; **dadurch gekennzeichnet, dass**
der Fluidweg im Gebrauch eine erste Anordnung, bei der der Teil des Fluidwegs in dem atraumatischen Ende im Wesentlichen mit Gas gefüllt ist, so dass das Ende eine verbesserte Echogenität hat, und eine zweite Anordnung hat, bei der der Teil des Fluidwegs in dem atraumatischen Ende im Wesentlichen mit Flüssigkeit gefüllt ist, so dass das Ende im Wesentlichen echotransparent ist.

2. System nach Anspruch 1, das ferner einen Katheter (104) mit einem Lumen aufweist, das zur Aufnahme des Führungsdrahts dimensioniert ist, so dass der Katheter am Führungsdraht entlang bewegbar ist.

3. System nach Anspruch 2, wobei der Katheter eine Sonde (106) zur Verwendung mit einem Ultraschallsystem enthält.

4. System nach Anspruch 1, wobei der Fluidweg ein mit einer Flüssigkeits- oder Gasquelle verbundener geschlossener Weg ist.

5. System nach Anspruch 1, das ferner eine Endkappe aufweist, die an einer Anwendungsseite des Führungsdrahts positioniert ist, wobei die Endkappe an einer Trägerröhre (200) angebracht ist.

6. System nach Anspruch 5, wobei die Endkappe eine Schulter hat, die an einem anwendungsseitigen Ende der Trägerröhre in Anlage ist.

7. System nach Anspruch 5, wobei die Endkappe aus einem Polymermaterial gefertigt ist, das bei Positionierung in einem Körperkanal im Wesentlichen echotransparent ist.

8. System nach Anspruch 1, wobei das Trägerelement ein sich verjüngendes Ende (218) enthält.

9. System nach Anspruch 1, wobei das Trägerelement aus einem Polymermaterial gefertigt ist.

10. System nach Anspruch 5, das ferner ein Loch in der Seite der Endkappe aufweist.

11. System nach Anspruch 5, wobei die Trägerröhre eine Spule oder eine Geflechtröhre ist.

12. System nach Anspruch 5, wobei die Trägerröhre aus Metall gefertigt ist.

13. System nach Anspruch 5, wobei das Trägerelement einen Außendurchmesser hat, der kleiner als der Durchmesser des Fluidwegs ist.

14. System nach Anspruch 5, wobei das Trägerelement einen Außendurchmesser hat, der im Wesentlichen der gleiche wie der des Fluidwegs ist.

15. System nach Anspruch 5, wobei das Trägerelement wenigstens teilweise im Fluidweg ist und zwischen einer ersten Stellung, in der ein Teil des Trägerelements im Endteil ist, und einer zweiten Stellung, in der das Trägerelement nicht im Endteil ist, bewegbar ist; und
wobei das Zurückziehen des Trägerelements die Echogenität des Endteils verändert, wenn der Endteil in einem Körperkanal positioniert ist.

## Revendications

1. Système destiné à être utilisé avec des procédures à ultrasons comprenant :
un fil-guide (102) doté d'une extrémité atraumatique et d'une partie d'une trajectoire de fluide au moins partiellement à l'intérieur de l'extrémité atraumatique ;
un élément de support (216) situé au moins partiellement à l'intérieur de la trajectoire de fluide et rétractable par rapport à celle-ci ; **caractérisé en ce que**
la trajectoire de fluide présente en service une première configuration dans laquelle la partie de la trajectoire de fluide à l'intérieur de l'extrémité atraumatique est sensiblement remplie d'un gaz de telle sorte que l'extrémité ait une échogénicité rehaussée et une seconde configuration dans laquelle la partie de la trajectoire de fluide à l'intérieur de l'extrémité atraumatique est sensiblement remplie de liquide de telle sorte que l'extrémité soit sensiblement échotransparente.

2. Système selon la revendication 1, comprenant en outre un cathéter (104) doté d'une lumière dimensionnée pour recevoir le fil-guide, de telle sorte que le cathéter puisse se déplacer le long du fil-guide.

3. Système selon la revendication 2, dans lequel le cathéter comporte une sonde (106) destinée à être utilisée avec un système à ultrasons.

4. Système selon la revendication 1, dans lequel la trajectoire de fluide est une trajectoire fermée reliée à une source de liquide ou de gaz.

5. Système selon la revendication 1, comprenant en outre un embout positionné au niveau d'un côté d'application du fil-guide, dans lequel l'embout est attaché à un tube de support (200).

6. Système selon la revendication 5, dans lequel l'embout comporte un épaulement qui se met en prise avec une extrémité côté application du tube de support.

7. Système selon la revendication 5, dans lequel l'embout est construit dans un matériau polymère qui est sensiblement échotransparent quand il est positionné dans un conduit corporel.

8. Système selon la revendication 1, dans lequel l'élément de support comporte une extrémité conique (218).

9. Système selon la revendication 1, dans lequel l'élément de support est construit dans un matériau polymère.

10. Système selon la revendication 5, comprenant en outre un orifice dans le côté de l'embout.

11. Système selon la revendication 5, dans lequel le tube de support est une bobine ou un tube tressé.

12. Système selon la revendication 5, dans lequel le tube de support est construit en métal.

13. Système selon la revendication 5, dans lequel l'élément de support a un diamètre externe inférieur au diamètre de la trajectoire de fluide.

14. Système selon la revendication 5, dans lequel l'élément de support a un diamètre externe sensiblement identique à celui de la trajectoire de fluide.

15. Système selon la revendication 5, dans lequel l'élément de support se trouve au moins partiellement à l'intérieur de la trajectoire de fluide et peut se déplacer entre une première position à laquelle une partie de l'élément de support se trouve à l'intérieur de la partie d'extrémité et une seconde position à laquelle l'élément de support ne se trouve pas à l'intérieur de la partie d'extrémité ; et
dans lequel la rétraction de l'élément de support modifie l'échogénicité de la partie d'extrémité quand la partie d'extrémité est positionnée à l'intérieur d'un conduit corporel.
